# EUROPEAN PATENT SPECIFICATION

(11) **EP 3 856 301 B1**
(45) Date of publication and mention of the grant of the patent: **26.02.2025**
(21) Application number: 19780178.0
(22) Date of filing: 23.09.2019
(51) Int. Cl.: A24F 7/00, A24F 40/30, A24F 40/40, A61M 11/04, A61M 15/06

(54) **AEROSOL DELIVERY DEVICE**
AEROSOLABGABEVORRICHTUNG
DISPOSITIF DE DISTRIBUTION D'AÉROSOL

(30) Priority: 24.09.2018 GB 201815471
(43) Date of publication of application: 04.08.2021
(60) Divisional application: 25151539.1
(73) Proprietor: Imperial Tobacco Limited, Bristol, BS3 2LL (GB)
(72) Inventor: AUSTIN, Andrew, Liverpool Merseyside L24 9HP (GB); SAJTOS, Tamas, Liverpool Merseyside L24 9HP (GB); TYLER, Andrew, Liverpool Merseyside L24 9HP (GB)
(74) Representative: Mewburn Ellis LLP
(86) International application number: PCT/EP2019/075489
(87) International publication number: WO 2020/064610

(56) References cited:
- EP-A1- 3 066 940
- WO-A1-2017/068099
- GB-A- 2 513 637
- US-A1- 2015 296 884
- US-A1- 2017 065 000
- US-A1- 2017 202 266
- US-A1- 2018 221 605
- US-A1- 2018 264 241

## Description

### Field of the Invention

The present disclosure relates to an aerosol delivery device and particularly, although not exclusively, to an aerosol delivery device comprising a support for maintaining an aerosol generator portion in a substantially central position.

### Background

A smoking-substitute device is an electronic device that permits the user to simulate the act of smoking by producing an aerosol mist or vapour that is drawn into the lungs through the mouth and then exhaled. The inhaled aerosol mist or vapour typically bears nicotine and/or other flavourings without the odour and health risks associated with traditional smoking and tobacco products. In use, the user experiences a similar satisfaction and physical sensation to those experienced from a traditional smoking or tobacco product, and exhales an aerosol mist or vapour of similar appearance to the smoke exhaled when using such traditional smoking or tobacco products.

One approach for a smoking substitute device is the so-called "vaping" approach, in which a vaporisable liquid, typically referred to (and referred to herein) as "e-liquid", is heated by a heating device to produce an aerosol vapour which is inhaled by a user. The e-liquid typically includes a base liquid as well as nicotine and/or flavourings. The resulting vapour therefore also typically contains nicotine and/or flavourings. The base liquid may include propylene glycol and/or vegetable glycerine.

A typical vaping smoking substitute device includes a mouthpiece, a power source (typically a battery), a tank for containing e-liquid, as well as a heating device. In use, electrical energy is supplied from the power source to the heating device, which heats the e-liquid to produce an aerosol (or "vapour") which is inhaled by a user through the mouthpiece.

Vaping smoking substitute devices can be configured in a variety of ways. For example, there are "closed system" vaping smoking substitute devices, which typically have a sealed tank and heating element. The tank is pre-filled with e liquid and is not intended to be refilled by an end user. One subset of closed system vaping smoking substitute devices include a main body which includes the power source, wherein the main body is configured to be physically and electrically coupled to a consumable including the tank and the heating element. In this way, when the tank of a consumable has been emptied, that consumable is disposed of. The main body can be reused by connecting it to a new, replacement, consumable. Another subset of closed system vaping smoking substitute devices are completely disposable, and intended for one-use only.

There are also "open system" vaping smoking substitute devices which typically have a tank that is configured to be refilled by a user. In this way the device can be used multiple times.

An example vaping smoking substitute device is the myblu^{™} e-cigarette. The myblu^{™} e cigarette is a closed system device which includes a main body and a consumable. The main body and consumable are physically and electrically coupled together by pushing the consumable into the main body. The main body includes a rechargeable battery. The consumable includes a mouthpiece, a sealed tank which contains e-liquid, as well as a heating device, which for this device is a heating filament coiled around a portion of a wick. The wick is partially immersed in the e-liquid, and conveys e-liquid from the tank to the heating filament. The device is activated when a microprocessor on board the main body detects a user inhaling through the mouthpiece. When the device is activated, electrical energy is supplied from the power source to the heating device, which heats e-liquid from the tank to produce a vapour which is inhaled by a user through the mouthpiece. A further exemplary smoking substitute device is described in GB2513637A. Additionally, US20180264241A1 proposes a methods and apparatuses to deliver biological altering substances through body cavities including ears, nose, mouth, anus and vagina, utilizing at least osmotic, olfactory and inhaling transfusion of active ingredients including a first reservoir configured to be disposed against sublingual surfaces and hold a first drug.

For a smoking substitute device it is desirable to deliver nicotine into the user's lungs, where it can be absorbed into the bloodstream. As explained above, in the so-called "vaping" approach, "e-liquid" is heated by a heating device to produce an aerosol vapour which is inhaled by a user. Many e-cigarettes also deliver flavour to the user, to enhance the experience. Flavour compounds are contained in the e-liquid that is heated. Heating of the flavour compounds may be undesirable as the flavour compounds are inhaled into the user's lungs. Toxicology restrictions are placed on the amount of flavour that can be contained in the e-liquid. This can result in some e-liquid flavours delivering a weak and underwhelming taste sensation to consumers in the pursuit of safety.

The present technology has been devised in light of the above considerations.

### Summary of the Invention

The invention is defined by the appended set of claims.

Optionally, the aerosol delivery device further comprises a mouthpiece, the mouthpiece comprising a mouthpiece aperture forming part of the air flow passage, wherein the support is located in the mouthpiece aperture.

In some embodiments, the support at least partially circumscribes the aerosol generator portion i.e. at least partially circumscribes at outer surface of the aerosol generator portion. Thus the support is an external support.

Advantageously, the support comprises ribs extending radially inwardly from a narrowing section of the air flow passage. The narrowing section of the air flow passage is typically substantially annular.

In some embodiments, the ribs contact and grip an outer surface of the aerosol generator portion. In some embodiments, the ribs may even penetrate the outer surface of the aerosol generator portion.

The ribs may be equally spaced around the circumference of the aerosol generator portion.

Conveniently, the support comprises three ribs (e.g. three equally spaced ribs) extending inwardly from the narrowing section of the air flow passage.

Optionally, the aerosol delivery device comprises a member, the member comprising the aerosol generator portion, wherein the member is configured to transfer the first aerosol precursor to the aerosol generator portion.

Advantageously, the member is formed of a second porous material, the member configured to wick the first aerosol precursor to the aerosol generator portion.

The air flow passage is configured to direct air past the aerosol generator portion to pick up the first aerosol precursor from the aerosol generator portion to form a first aerosol i.e. the aerosol generator portion is a passive aerosol generator portion that generates the first aerosol without the application of heat.

The aerosol delivery device further comprises a storage for storing the first aerosol precursor.

Optionally, the storage comprises a reservoir, the reservoir formed of a first porous material.

The storage comprises a tank configured to store the first aerosol precursor as a free liquid.

Conveniently, the first aerosol is sized to inhibit pulmonary penetration, and the first aerosol is transmissible within at least one of a mammalian oral cavity and a mammalian nasal cavity.

Advantageously, the aerosol delivery device is a consumable for a smoking substitute device.

Conveniently, the aerosol delivery device comprises a second aerosol generator, the second aerosol generator configured to produce a second aerosol from a second aerosol precursor, wherein the second aerosol is sized for pulmonary penetration.

Optionally, the second aerosol generator is configured to heat the second aerosol precursor to form the second aerosol.

Conveniently, the second aerosol generator is located so as to be upstream of the first aerosol generator in use.

Advantageously, the first aerosol precursor comprises a flavour component.

Conveniently, the second aerosol precursor comprises an active component.

Optionally, the active component is nicotine.

The invention includes the combination of the aspects and preferred features described except where such a combination is clearly impermissible or expressly avoided.

### Summary of the Figures

So that the invention may be understood, and so that further aspects and features thereof may be appreciated, embodiments illustrating the principles of the invention will now be discussed in further detail with reference to the accompanying figures, in which:
**Fig. 1** shows a schematic drawing of a smoking substitute system;
**Fig. 2** shows a schematic drawing of a smoking substitute system;
**Fig. 3** shows a schematic drawing of a smoking substitute system;
**Fig. 4** shows a schematic drawing of a smoking substitute system;
**Fig. 5** shows a cutaway view of a consumable;
**Fig. 6** shows a cross-sectional view of a flavour pod portion of a consumable;
**Fig. 7a** shows a top view of a flavour pod portion of a consumable;
**Fig. 7b** shows a perspective cross-sectional view of a flavour pod portion of a consumable; and
**Fig. 7c** shows a top view of a part of a flavour pod portion of a consumable.

### Detailed Description of the Invention

Aspects and embodiments of the present invention will now be discussed with reference to the accompanying figures. Further aspects and embodiments will be apparent to those skilled in the art.

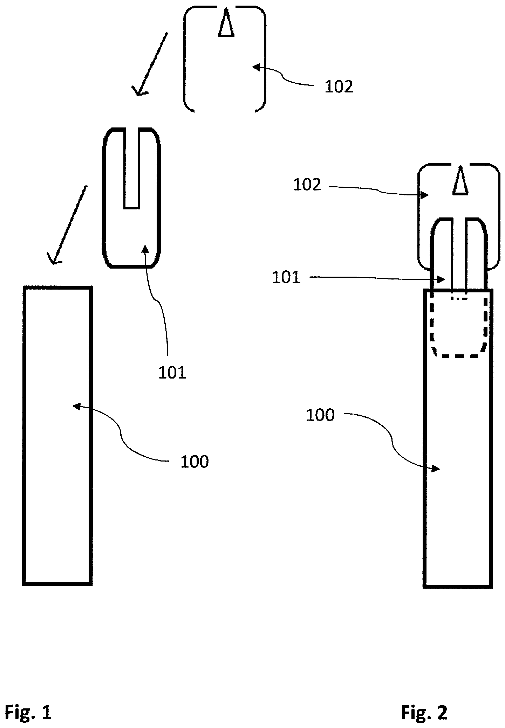

Referring to Figures 1 and 2, there is shown a smoking substitute system comprising a smoking substitute device 100. In this example, the substitute smoking system comprises a cartomiser 101 and a flavour pod 102. The cartomiser 101 may engage with the smoking substitute device 100 via a push-fit engagement, a screw-thread engagement, or a bayonet fit, for example. A cartomiser may also be referred to as a "pod". The smoking substitute system may be an aerosol delivery device according to the present disclosure.

The flavour pod 102 is configured to engage with the cartomiser 101 and thus with the substitute smoking device 100. The flavour pod 102 may engage with the cartomiser 101 via a push-fit engagement, a screw-thread engagement, or a bayonet fit, for example. Fig. 2 illustrates the cartomiser 101 engaged with the substitute smoking device 100, and the flavour pod 102 engaged with the cartomiser 101. As will be appreciated, in this example, the cartomiser 101 and the flavour pod 102 are distinct elements. Each of the cartomiser 101 and the flavour pod may be an aerosol delivery device according to the present disclosure.

As will be appreciated from the following description, the cartomiser 101 and the flavour pod 102 may alternatively be combined into a single component that implements the functionality of the cartomiser 101 10 and flavour pod 102. Such a single component may also be an aerosol delivery device according to the present disclosure.

In other examples, the cartomiser may be absent, with only a flavour pod 102 present.

A "consumable" component may mean that the component is intended to be used once until exhausted, and then disposed of as waste or returned to a manufacturer for reprocessing.

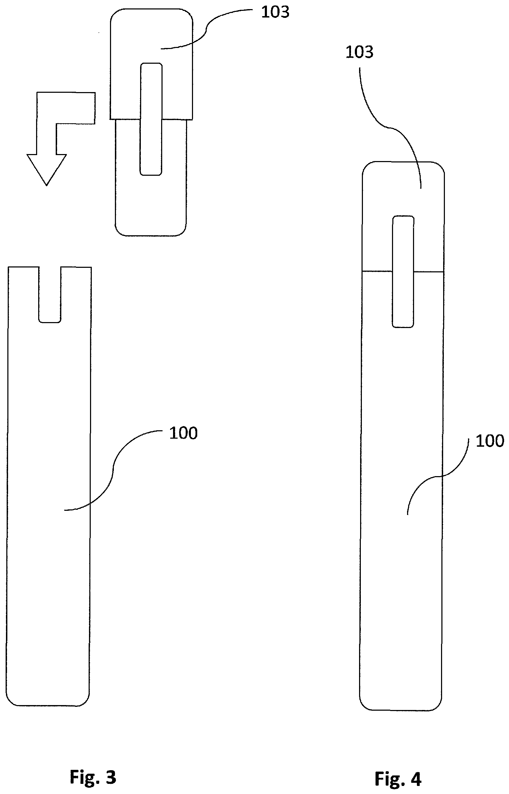

Referring to Figures 3 and 4, there is shown a smoking substitute system comprising a smoking substitute device 100 and a consumable 103. The consumable 103 combines the functionality of the cartomiser 101 and the flavour pod 102. In Figure 3, the consumable 103 and the smoking substitute device 100 are shown separated from one another. In Figure 4, the consumable 103 and the smoking substitute device 100 are engaged with each other.

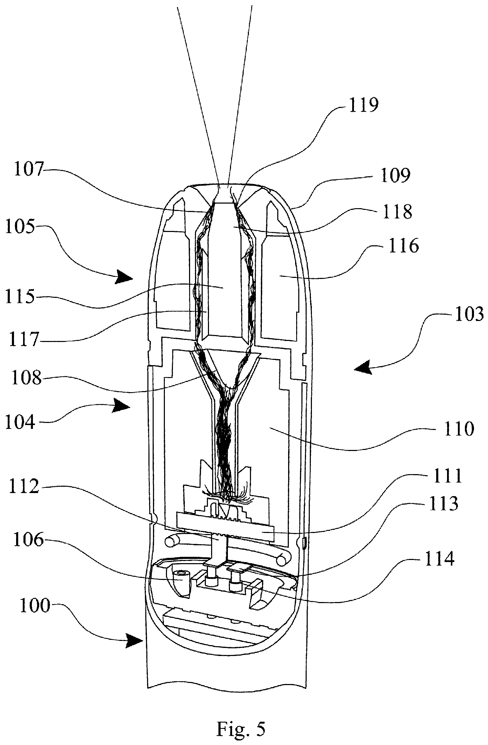

Referring to Figure 5, there is shown a consumable 103 engaged with a smoking substitute device 100 via a push-fit engagement. The consumable 103 may be considered to have two portions - a cartomiser portion 104 and a flavour pod portion 105, both of which are located within a single component (as in Figures 3 and 4).

The consumable 103 includes an upstream airflow inlet 106 and a downstream airflow outlet 107. In other examples a plurality of inlets and/or outlets are included. Between and fluidly connecting the inlet 106 and the outlet 107 there is an airflow passage 108. The outlet 107 is located at the mouthpiece 109 of the consumable 103, and is formed by a mouthpiece aperture.

As above, the consumable 103 includes a flavour pod portion 105. The flavour pod portion 105 is configured to generate a first (flavour) aerosol for output from the outlet 107 of the mouthpiece 109 of the consumable 103. The flavour pod portion 105 of the consumable 103 includes a member 115. The member 115 acts as a passive aerosol generator (i.e. an aerosol generator which does not use heat to form the aerosol, also referred to as a "first aerosol generator" in this example), and is formed of a porous material. The member 115 comprises a supporting portion 117, which is located inside a housing, and an aerosol generator portion 118, which is located in the airflow passage 108. In this example, the aerosol generator portion 118 is a porous nib.

A first storage 116 (in this example a tank) for storing a first aerosol precursor (i.e. a flavour liquid) is fluidly connected to the member 115. The porous nature of the member 115 means that flavour liquid from the first storage 116 is drawn into the member 115. As the first aerosol precursor in the member 115 is depleted in use, further flavour liquid is drawn from the first storage 116 into the member 115 via a wicking action.

As described above, the aerosol generator portion 118 is located within the airflow passage 108 through the consumable 103. The aerosol generator portion 118 therefore constricts or narrows the airflow passage 108. The aerosol generator portion 118 occupies some of the area of the airflow passage, resulting in constriction of the airflow passage 108. The airflow passage 108 is narrowest adjacent to the aerosol generator portion 118. Since the constriction results in increase air velocity and corresponding reduction in air pressure at the aerosol generator portion 118, the constriction is a Venturi aperture 119.

The cartomiser portion 104 of the consumable 103 includes a second storage 110 (in this example a tank) for storing a second aerosol precursor (i.e. e-liquid, which may contain nicotine). Extending into the second storage 110 is a wick 111. The wick 111 is formed from a porous wicking material (e.g. a polymer) that draws second aerosol precursor from the second storage 110 into a central region of the wick 111 that is located outside the e-liquid storage tank 110.

A heater 112 is a configured to heat the central region of the wick 111. The heater 112 includes a resistive heating filament that is coiled around the central region of the wick 111. The wick 111, the heater 112 and the e-liquid storage tank 110 together act as an active aerosol generator (i.e. an aerosol generator which uses heat to form the aerosol, referred to as a "second aerosol generator" in this example).

As described above, the first and second aerosol generators are both at least partially located within the airflow passage 108, with the first aerosol generator downstream (with respect to air flow in use) of the second aerosol generator.

So that the consumable 103 may be supplied with electrical power for activation of the heater 112, the consumable 103 includes a pair of consumable electrical contacts 113. The consumable electrical contacts 113 are configured for electrical connection to a corresponding pair of electrical supply contacts 114 in the smoking substitute device 100. The consumable electrical contacts 113 are electrically connected to the electrical supply contacts 114 when the consumable 103 is engaged with the smoking substitute device 100. The smoking substitute device 100 includes an electrical power source (not shown), for example a battery.

In use, a user draws (or "sucks", or "pulls") on the mouthpiece 109 of the consumable 103, which causes a drop in air pressure at the outlet 107, thereby generating air flow through the inlet 106, along the airflow passage 108, out of the outlet 107 and into the user's mouth.

When the heater 112 is activated (by passing an electric current through the heating filament in response to the user drawing on the mouthpiece 109) the e-liquid located in the wick 111 adjacent to the heating filament is heated and vaporised to form a vapour. The vapour condenses to form the second aerosol within the airflow passage 108. Accordingly, the second aerosol is entrained in an airflow along the airflow flow passage 108 to the outlet 107 and ultimately out from the mouthpiece 109 for inhalation by the user when the user 10 draws on the mouthpiece 109.

The substitute smoking device 100 supplies electrical current to the consumable electrical contacts 113. This causes an electric current flow through the heating filament of the heater 112 and the heating filament heats up. As described, the heating of the heating filament causes vaporisation of the e-liquid in the wick 111 to form the second aerosol.

As the air flows up through the airflow passage 108, it encounters the aerosol generator portion 118. The constriction of the airflow passage 108 caused by the aerosol generator portion 118 results in an increase in air velocity and corresponding decrease in air pressure in the airflow in the vicinity of the porous surface 118 of the aerosol generator portion 115. The corresponding low pressure region causes the generation of the first (flavour) aerosol from the porous surface 118 of the aerosol generator portion 118. The first (flavour) aerosol is entrained into the airflow and ultimately is output from the outlet 107 of the consumable 103 and thus from the mouthpiece 109 into the user's mouth.

The first aerosol is sized to inhibit pulmonary penetration. The first aerosol is formed of particles with a mass median aerodynamic diameter that is greater than or equal to 15 microns, in particular, greater than 30 microns, more particularly greater than 50 microns, yet more particularly greater than 60 microns, and even more particularly greater than 70 microns.

The first aerosol is sized for transmission within at least one of a mammalian oral cavity and a mammalian nasal cavity. The first aerosol is formed by particles having a maximum mass median aerodynamic diameter that is less than 300 microns, in particular less than 200 microns, yet more particularly less than 100 microns. Such a range of mass median aerodynamic diameter will produce aerosols which are sufficiently small to be entrained in an airflow caused by a user drawing air through the flavour element and to enter and extend through the oral and or nasal cavity to activate the taste and/or olfactory receptors.

The second aerosol generated is sized for pulmonary penetration (i.e. to deliver an active ingredient such as nicotine to the user's lungs). The second aerosol is formed of particles having a mass median aerodynamic diameter of less than or equal to 10 microns, preferably less than 8 microns, more preferably less than 5 microns, yet more preferably less than 1 micron. Such sized aerosols tend to penetrate into a human user's pulmonary system, with smaller aerosols generally penetrating the lungs more easily. The second aerosol may also be referred to as a vapour.

The size of aerosol formed without heating is typically smaller than that formed by condensation of a vapour.

As a brief aside, it will be appreciated that the mass median aerodynamic diameter is a statistical measurement of the size of the particles/droplets in an aerosol. That is, the mass median aerodynamic diameter quantifies the size of the droplets that together form the aerosol. The mass median aerodynamic diameter may be defined as the diameter at which 50% of the particles/droplets by mass in the aerosol are larger than the mass median aerodynamic diameter and 50% of the particles/droplets by mass in the aerosol are smaller than the mass median aerodynamic diameter. The "size of the aerosol", as may be used herein, refers to the size of the particles/droplets that are comprised in the particular aerosol.

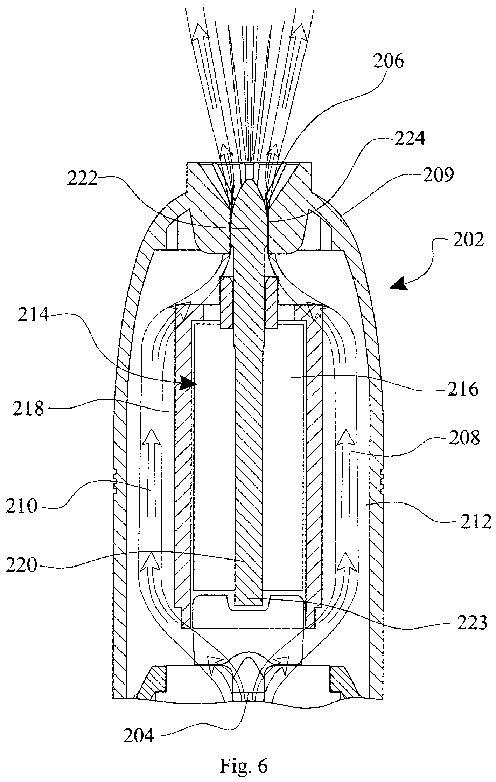

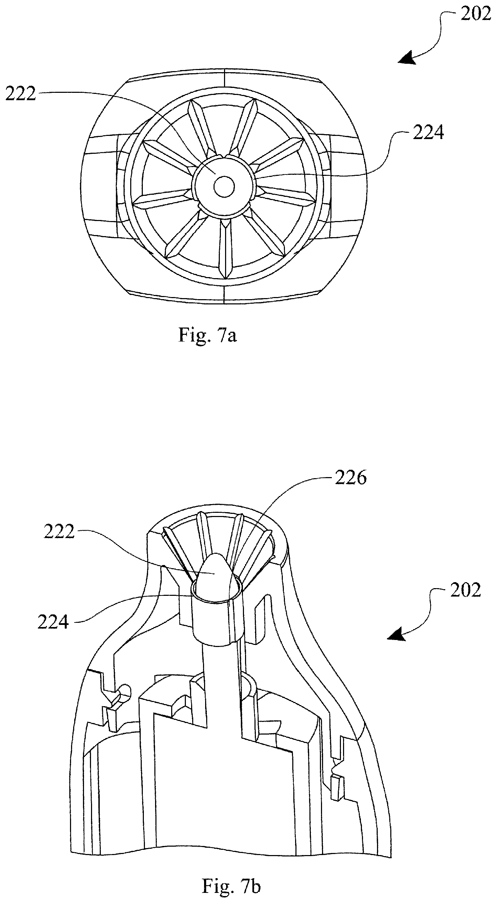

Referring to Fig. 6, Fig. 7a and Fig. 7b, there is shown a flavour pod portion 202 of a consumable, the consumable providing an aerosol delivery device in accordance with the invention. For clarity, many reference numerals are omitted from Fig. 7a and 7b. The consumable further comprises a cartomiser portion (not shown in Fig. 6) having all of the features of the cartomiser portion 104 described above with respect to Fig. 5. However, in other examples, the consumable does not comprise the cartomiser portion, and provides only flavour to the user.

The flavour pod portion 202 comprises an upstream (i.e. upstream with respect to flow of air in use) inlet 204 and a downstream (i.e. downstream with respect to flow of air in use) outlet 206. Between and fluidly connecting the inlet 204 and the outlet 206 the flavour pod portion 204 comprises an airflow passage 208. The airflow passage 208 comprises a first airflow branch 210 and a second airflow branch 212, each of the first airflow branch 210 and the second airflow branch 212 fluidly connecting the inlet 204 and the outlet 206. In other examples the airflow passage 208 may have an annular shape. The outlet 206 is located at the mouthpiece 209 of the consumable 103, and is also referred to as a mouthpiece aperture 206.

The flavour pod portion 202 comprises a storage 214, which stores a first aerosol precursor. The storage 214 comprises a reservoir 216 located within a chamber 218. The reservoir 216 is formed of a first porous material.

The flavour pod portion 202 comprises a member 220, which comprises an aerosol generator portion 222 and a supporting portion 223. The aerosol generator portion 222 is located at a downstream end (an upper end in Fig. 6) of the member 220, while the supporting portion 223 makes up the rest of the member 220. The supporting portion 223 is elongate and substantially cylindrical. The aerosol generator portion 222 is bulb-shaped, and comprises a portion which is wider than the supporting portion 223. The aerosol generator portion 222 tapers to a tip at a downstream end of the aerosol generator portion 222.

The member 220 extends into and through the storage 214. The member 220 is in contact with the reservoir 216. More specifically, the supporting portion 223 extends into and through the storage 204 and is in contact with the reservoir 216. The member 220 is located in a substantially central position within the reservoir 216 and is substantially parallel to a central axis of the consumable. The member 220 is formed of a second porous material.

The first and second airflow branches 210, 212 are located on opposite sides of the member 220. Additionally, the first and second airflow branches 210, 212 are located on opposite sides of the reservoir 216. The first and second airflow branches 210, 212 branch in a radial outward direction (with respect to the central axis of the consumable 200) downstream of the inlet 204 to reach the opposite sides of the reservoir 216.

The aerosol generator portion 222 is located in the airflow passage 208 downstream of the first and second airflow branches 210, 212. The first and second airflow branches 210, 212 turn in a radially inward direction to merge at the member 220, at a point upstream of the aerosol generator portion 222.

The aerosol generator portion 222 is located in a narrowing section 224 of the airflow passage 208. The narrowing section 224 is downstream of the point at which the first and second airflow branches 210 212 merge, but upstream of the mouthpiece aperture 207. The mouthpiece aperture 207 flares outwardly in the downstream direction, such that a width of the mouthpiece aperture 207 increases in the downstream direction.

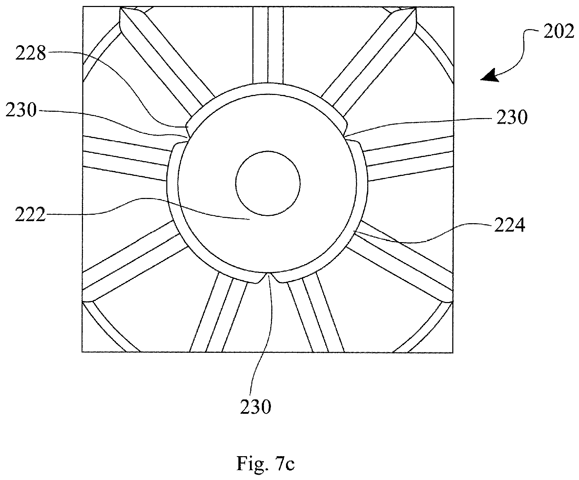

Referring to Fig. 7a, the air flow passage 208 has a cross-sectional area of at most 2.0 mm² at the aerosol generator portion (i.e. at the narrowing section 224, as indicated in Fig. 7a, and excluding the support described below with respect to Fig. 7c). This is the area of the annular portion at which the aerosol generator portion is positioned, downstream of the point at which the first and second airflow branches 210, 212 merge. More specifically, the air flow passage 208 has a cross-sectional area of at most 1.8 mm² at the aerosol generator portion. More specifically, the air flow passage 208 has a cross-sectional area of at most 1.6 mm² at the aerosol generator portion. More specifically, the air flow passage 208 has a cross-sectional area of at most 1.4 mm² at the aerosol generator portion. More specifically, the air flow passage 208 has a cross-sectional area of at most 1.2 mm² at the aerosol generator portion.

The air flow passage 208 has a cross-sectional area of at least 0.2 mm² at the aerosol generator portion (again at the narrowing section 224, as indicated in Fig. 7a). More specifically, the air flow passage 208 has a cross-sectional area of at least 0.4 mm² at the aerosol generator portion. More specifically, the air flow passage 208 has a cross-sectional area of at least 0.6 mm² at the aerosol generator portion. More specifically, the air flow passage 208 has a cross-sectional area of at least 0.8 mm² at the aerosol generator portion. More specifically, the air flow passage 208 has a cross-sectional area of substantially 1.0 mm² at the aerosol generator portion.

In this example, the areas of the air flow passage 208 described here relate to the narrowest area through which air flows past the aerosol generator portion. However, since there is little variation in the width of the aerosol generator portion 222 in the narrowing section 224, this area is substantially constant throughout the narrowing section 224.

Referring to Fig. 7b, the aerosol generator portion 222 has an atomising area 226 of more than 10 mm². The atomising area may be defined as the surface area of the portion from which substantial first aerosol is generated, which in this example is the surface area of the part of the aerosol generator portion which is located in the narrowing section 224. The atomising area may be defined as the surface area from which 95% of the first aerosol is generated.

More specifically, the atomising area 226 is at least 16 mm². More specifically, the atomising area 226 is at least 18 mm². More specifically, the atomising area 226 is at least 20 mm². More specifically, the atomising area 226 is at least 20 mm².

The atomising area 226 is not more than 36 mm². More specifically, the atomising area 226 is not more than 30 mm². More specifically, the atomising area 226 is not more than 28 mm². More specifically, the atomising area 226 is not more than 26 mm². More specifically, the atomising area 226 is not more than 24 mm².

More specifically, the atomising area 226 is substantially 22 mm².

Referring to Fig. 7c, the flavour pod portion 202 further comprises a support 228. The support 228 comprises ribs 230 extending inwardly from the narrowing section 224. The support 228 comprises three ribs 230 extending inwardly from the narrowing section 224 of the air flow passage 208. The ribs 230 are substantially equi-spaced around the narrowing section 224. Apart from the support 228, the narrowing section 224 has a generally annular shape.

The ribs 230 contact the aerosol generator portion 222. The ribs 230 pierce an outer surface of the aerosol generator portion 222 to thereby grip the aerosol generator portion 222.

In use, when a user draws on the mouthpiece 209, air flow is generated through the air flow passage 208. Air (comprising the second aerosol from the cartomiser portion as explained above with respect to Fig. 5) flows through the inlet 204 before the air flow splits to flow through the first and second airflow branches 210, 212. Further downstream, the first and second airflow branches 210, 212 provide inward airflow towards the member 220 and the aerosol generator portion 222.

As air flows past the aerosol generator portion in the narrowing section 224, the velocity of the air increases, resulting in a drop in air pressure. This means that the air picks up the first aerosol precursor from the aerosol generator portion 222 to form the first aerosol. The first aerosol has the particle size and other properties described above with respect to Fig. 5.

The air picks up more than 1.0 mg total particle mass (TPM) of first aerosol per delivery event. More specifically, the air picks up more than 1.5 mg TPM of first aerosol per delivery event. More specifically, the air picks up more than 2.0 mg TPM of first aerosol per delivery event. More specifically, the air picks up more than 2.5 mg TPM of first aerosol per delivery event. More specifically, the air picks up more than 3.0 mg TPM of first aerosol per delivery event.

The air picks up less than 20.0 mg TPM of first aerosol per delivery event. More specifically, the air picks up less than 13.0 mg TPM of first aerosol per delivery event. More specifically, the air picks up less than 8.0 mg TPM of first aerosol per delivery event. More specifically, the air picks up less than 6.0 mg TPM of first aerosol per delivery event. More specifically, the air picks up less than 4.0 mg TPM of first aerosol per delivery event.

First aerosol of the TPM values described above may be picked up when the aerosol generator portion 223 is saturated, which may be when the storage 214 is full. A delivery event may refer to a typical single draw on the mouthpiece 209 by a user.

In a delivery event, the user may inhale on the mouthpiece 209 such that a flow rate of between 1.0 and 4.0 litres/minute is effected in the airflow passage 208. The user may inhale on the mouthpiece 209 such that a flow rate of substantially 2.5 litres/minute is effected in the airflow passage 208. Such a flow rate may result in a velocity of substantially 40 m/s in the narrowing section 224.

The delivery event may have a duration (i.e. the period for which the user inhales on the mouthpiece 209) of between 1.0 and 4.0 seconds. The delivery event may have a duration of between 1.5 and 2.5 seconds, and in some examples substantially 2.0 seconds.

As the first aerosol precursor is picked up by the air, the member 220 transfers further first aerosol precursor from the storage 214 to the aerosol generator portion 222. More specifically, the member 220 wicks the first aerosol precursor from the storage 214 to the aerosol generator portion 223.

In other examples, the storage 214 comprises a tank containing the first aerosol precursor as free liquid, rather than the reservoir 216 and the chamber 218. In such examples, the member 220 still extends into the tank to transfer first aerosol precursor from the tank to the aerosol generator portion 223.

The support 228 maintains the aerosol generator portion 222 in a substantially central position in the narrowing section 224.

In other examples, the storage 214 comprises a tank containing the first aerosol precursor as free liquid, rather than the reservoir 216 and the chamber 218. In such examples, the member 220 still extends into the tank to transfer first aerosol precursor from the tank to the aerosol generator portion 223.

## Claims

1. An aerosol delivery device (100) comprising:
an aerosol generator portion (118) configured to receive a first aerosol precursor, wherein the aerosol generator portion (118) is a passive aerosol generator portion (118);
an air flow passage (208) configured to direct air past the aerosol generator portion (118) to pick up the first aerosol precursor from the aerosol generator portion (118) to form a first aerosol; and
a support (228) for maintaining the aerosol generator portion (118) in a substantially central position in the air flow passage (208);
**characterised in that** the device further comprises a storage (214) for storing the first aerosol precursor wherein the storage (214) comprises a tank configured to store the first aerosol precursor as a free liquid.

2. An aerosol delivery device (100) according to claim 1, and further comprising a mouthpiece (109), the mouthpiece (109) comprising a mouthpiece aperture (206) forming part of the air flow passage (208), wherein the support (228) is located in the mouthpiece aperture (206).

3. An aerosol delivery device (100) according to claim 2, wherein the support (228) comprises ribs extending inwardly from a narrowing section of the air flow passage (208).

4. An aerosol delivery device (100) according to claim 3, wherein the support (228) comprises three ribs extending inwardly from the narrowing section air flow passage (208).

5. An aerosol delivery device (100) according to any preceding claim, and further comprising a member (115), the member (115) comprising the aerosol generator portion (118), wherein the member (115) is configured to transfer the first aerosol precursor to the aerosol generator portion (118).

6. An aerosol delivery device (100) according to claim 5, wherein the member (115) is formed of a second porous material, the member (115) configured to wick the first aerosol precursor to the aerosol generator portion (118).

7. An aerosol delivery device (100) according to any one of the preceding claims, wherein the first aerosol is sized to inhibit pulmonary penetration, and the first aerosol is transmissible within at least one of a mammalian oral cavity and a mammalian nasal cavity.

8. An aerosol delivery device (100) according to any preceding claim, wherein the aerosol delivery device (100) is a consumable for a smoking substitute device.

9. An aerosol delivery device (100) according to any preceding claim and further comprising a second aerosol generator, the second aerosol generator configured to produce a second aerosol from a second aerosol precursor, wherein the second aerosol is sized for pulmonary penetration.

10. An aerosol delivery device (100) according to claim 9, wherein the second aerosol generator is configured to heat the second aerosol precursor to form the second aerosol.

11. An aerosol delivery device (100) according to claim 9 or claim 10, wherein the second aerosol generator is located so as to be upstream of the first aerosol generator in use.

## Patentansprüche

1. Aerosolzufuhrvorrichtung (100), umfassend:
einen Aerosolgeneratorabschnitt (118), der dazu ausgelegt ist, einen ersten Aerosolvorläufer aufzunehmen, wobei der Aerosolgeneratorabschnitt (118) ein passiver Aerosolgeneratorabschnitt (118) ist;
ein Luftströmungsdurchgang (208), der dazu ausgelegt ist, Luft am Aerosolgeneratorabschnitt (118) vorbeizuleiten, um den ersten Aerosolvorläufer aus dem Aerosolgeneratorabschnitt (118) mitzureißen, um ein erstes Aerosol auszubilden; und
eine Halterung (228) zum Halten des Aerosolgeneratorabschnitts (118) in einer im Wesentlichen zentralen Position im Luftströmungsdurchgang (208);
**dadurch gekennzeichnet, dass** die Vorrichtung ferner einen Speicher (214) zum Speichern des ersten Aerosolvorläufers umfasst, wobei der Speicher (214) einen Behälter umfasst, der dazu ausgelegt ist, den ersten Aerosolvorläufer als freie Flüssigkeit zu speichern.

2. Aerosolzufuhrvorrichtung (100) nach Anspruch 1 und ferner umfassend ein Mundstück (109), wobei das Mundstück (109) eine Mundstücköffnung (206) umfasst, die einen Teil des Luftströmungsdurchgangs (208) bildet, wobei die Halterung (228) in der Mundstücköffnung (206) angeordnet ist.

3. Aerosolzufuhrvorrichtung (100) nach Anspruch 2, wobei die Halterung (228) Rippen umfasst, die sich von einem sich verschmälernden Abschnitt des Luftströmungsdurchgangs (208) nach innen erstrecken.

4. Aerosolzufuhrvorrichtung (100) nach Anspruch 3, wobei die Halterung (228) drei Rippen umfasst, die sich vom sich verschmälernden Abschnitt des Luftströmungsdurchgangs (208) nach innen erstrecken.

5. Aerosolzufuhrvorrichtung (100) nach einem der vorangegangenen Ansprüche und ferner umfassend ein Element (115), wobei das Element (115) den Aerosolgeneratorabschnitt (118) umfasst, wobei das Element (115) dazu ausgelegt ist, den ersten Aerosolvorläufer zum Aerosolgeneratorabschnitt (118) zu befördern.

6. Aerosolzufuhrvorrichtung (100) nach Anspruch 5, wobei das Element (115) aus einem zweiten porösen Material ausgebildet ist, wobei das Element (115) dazu ausgelegt ist, den ersten Aerosolvorläufer zum Aerosolgeneratorabschnitt (118) mittels Dochtwirkung zu bewegen.

7. Aerosolzufuhrvorrichtung (100) nach einem der vorangegangenen Ansprüche, wobei das erste Aerosol bemessen ist, um Lungenpenetration zu hemmen, und das erste Aerosol innerhalb zumindest eines aus einer Säugetiermundhöhle und einer Säugetiernasenhöhle übertragbar ist.

8. Aerosolzufuhrvorrichtung (100) nach einem der vorangegangenen Ansprüche, wobei die Aerosolzufuhrvorrichtung (100) eine Verbrauchsware für eine Rauchersatzvorrichtung ist.

9. Aerosolzufuhrvorrichtung (100) nach einem der vorangegangenen Ansprüche und ferner umfassend einen zweiten Aerosolgenerator, wobei der zweite Aerosolgenerator dazu ausgelegt ist, ein zweites Aerosol aus einem zweiten Aerosolvorläufer zu erzeugen, wobei das zweite Aerosol für die Lungenpenetration bemessen ist.

10. Aerosolzufuhrvorrichtung (100) nach Anspruch 9, wobei der zweite Aerosolgenerator dazu ausgelegt ist, den zweiten Aerosolvorläufer zu erhitzen, um das zweite Aerosol zu bilden.

11. Aerosolzufuhrvorrichtung (100) nach Anspruch 9 oder 10, wobei der zweite Aerosolgenerator so angeordnet ist, dass er in Verwendung stromaufwärts zum ersten Aerosolgenerator ist.

## Revendications

1. Dispositif de distribution d'aérosol (100) comprenant :
une partie de générateur d'aérosol (118) configurée pour recevoir un premier précurseur d'aérosol, dans lequel la partie de générateur d'aérosol (118) est une partie de générateur d'aérosol passive (118) ;
un passage d'écoulement d'air (208), configuré pour diriger de l'air au-delà de la partie de générateur d'aérosol (118) afin de recueillir le premier précurseur d'aérosol à partir de la partie de générateur d'aérosol (118) pour former un premier aérosol ; et
un support (228) pour maintenir la partie de générateur d'aérosol (118) dans une position sensiblement centrale dans le passage d'écoulement d'air (208) ;
**caractérisé en ce que** le dispositif comprend en outre un stockage (214) pour stocker le premier précurseur d'aérosol, dans lequel le stockage (214) comprend un réservoir configuré pour stocker le premier précurseur d'aérosol sous la forme d'un liquide libre.

2. Dispositif de distribution d'aérosol (100) selon la revendication 1, et comprenant en outre un embout buccal (109), l'embout buccal (109) comprenant une ouverture d'embout buccal (206) faisant partie du passage d'écoulement d'air (208), dans lequel le support (228) est situé dans l'ouverture d'embout buccal (206).

3. Dispositif de distribution d'aérosol (100) selon la revendication 2, dans lequel le support (228) comprend des nervures s'étendant vers l'intérieur depuis une section de rétrécissement du passage d'écoulement d'air (208).

4. Dispositif de distribution d'aérosol (100) selon la revendication 3, dans lequel le support (228) comprend trois nervures s'étendant vers l'intérieur depuis le passage d'écoulement d'air de section de rétrécissement (208).

5. Dispositif de distribution d'aérosol (100) selon l'une quelconque des revendications précédentes, et comprenant en outre un élément (115), l'élément (115) comprenant la partie de générateur d'aérosol (118), dans lequel l'élément (115) est configuré pour transférer le premier précurseur d'aérosol vers la partie de générateur d'aérosol (118).

6. Dispositif de distribution d'aérosol (100) selon la revendication 5, dans lequel l'élément (115) est formé d'un second matériau poreux, l'élément (115) étant configuré afin d'évacuer le premier précurseur d'aérosol vers la partie de générateur d'aérosol (118).

7. Dispositif de distribution d'aérosol (100) selon l'une quelconque des revendications précédentes, dans lequel le premier aérosol est dimensionné pour inhiber une pénétration pulmonaire, et le premier aérosol peut être transmis à l'intérieur d'au moins une parmi une cavité buccale de mammifère et une cavité nasale de mammifère.

8. Dispositif de distribution d'aérosol (100) selon l'une quelconque des revendications précédentes, dans lequel le dispositif de distribution d'aérosol (100) est un consommable pour un dispositif à fumer de substitution.

9. Dispositif de distribution d'aérosol (100) selon l'une quelconque des revendications précédentes et comprenant en outre un second générateur d'aérosol, le second générateur d'aérosol étant configuré pour produire un second aérosol à partir d'un second précurseur d'aérosol, dans lequel le second aérosol est dimensionné pour une pénétration pulmonaire.

10. Dispositif de distribution d'aérosol (100) selon la revendication 9, dans lequel le second générateur d'aérosol est configuré pour chauffer le second précurseur d'aérosol afin de former le second aérosol.

11. Dispositif de distribution d'aérosol (100) selon la revendication 9 ou la revendication 10, dans lequel le second générateur d'aérosol est situé de manière à être en amont du premier générateur d'aérosol en utilisation.
